(19) Europäisches Patentamt
European Patent Office
Office européen des brevets

(11) **EP 4 278 946 A1**

(12) **EUROPEAN PATENT APPLICATION**

(43) Date of publication:
**22.11.2023 Bulletin 2023/47**

(21) Application number: **22174702.5**

(22) Date of filing: **20.05.2022**

(51) International Patent Classification (IPC):
*A61B 1/00* (2006.01)     *H02K 26/00* (2006.01)
*H02K 41/03* (2006.01)    *A61B 34/00* (2016.01)
*H02K 11/215* (2016.01)   *H02K 11/25* (2016.01)
*H02K 11/27* (2016.01)    *H02K 11/30* (2016.01)

(52) Cooperative Patent Classification (CPC):
**H02K 26/00; A61B 1/00158;** A61B 34/73;
H02K 11/215; H02K 11/25; H02K 11/27;
H02K 11/30; H02K 41/031; H02K 2201/18

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **UNIVERSITEIT TWENTE
7522 NB Enschede (NL)**
• **Korea Institute of Medical Microrobotics
Gwangju 61011 (KR)**
• **Industry Foundation of Chonnam National
University
Gwangju 61186 (KR)**

(72) Inventors:
• **Alaa Eldin Adel Mohamed Mahmoud, Sadek
7522NB Enschede (NL)**
• **Mina Maged Micheal, Farag
7522NB Enschede (NL)**
• **Sarthak, Misra
7522NB Enschede (NL)**
• **Islam S. M., Khalil
7522NB Enschede (NL)**
• **Park, Jong Oh
10211 Goyang-si (KR)**
• **Kim, Chang Sei
61011 Gwangju (KR)**

(74) Representative: **Arnold & Siedsma
Bezuidenhoutseweg 57
2594 AC The Hague (NL)**

(54) **SPHERICAL ELECTROMAGNETIC ACTUATOR AND METHOD FOR CONTROLLING A MAGENTIC FIELD THEREOF**

(57)     The invention relates to a spherical electromagnetic actuator. The spherical electromagnetic actuator including a spherical permanent magnet having a center region, a housing comprising a chamber enclosing the spherical magnet and having an inlet for receiving a fluid. The actuator further including three electromagnetic coil assemblies with each coil assembly having at least one coil and a virtual symmetry axis, and wherein the three electromagnetic coil assemblies are arranged such that the three respective symmetry axes intersect at a center region the spherical permanent magnet. The housing is arranged such that in operation the fluid causes the spherical magnet to levitate. And, in operation, the three electromagnetic coil assemblies provide a superpositioned magnetic field manipulating the orientation of the spherical magnet. The invention further relates to a method for controlling the magnetic field of at least one spherical magnetic actuator.

FIG. 2

## Description

The invention relates to an electromagnetic actuator for controlling a microrobot or endoscopic capsule by means of an electromagnetic field. In particular, the invention relates to a spherical electromagnetic actuator for generating a time-varying rotating magnetic field that facilitates manipulation of a magnetically propelled helical microrobot or helical endoscopic capsule. The invention further relates to a method for controlling a magnetic field of a spherical magnetic actuator.

The present disclosure has been made according to project number HI19C0642 under the support of the Ministry of Health and Welfare, the research management institution for the above project is the Korea Health Industry Development Institute, the title of the research business is "Development of Technology and Commercialization for Medical Micro-robots", the title of the research project is "Common Basis Technology Development Center for Commercialization of Medical Micro-robots", the host organization thereof is the Korea Institute of Medical Microrobotics, and the research period thereof is June 12, 2019 to December 31, 2022.

## BACKGROUND

For minimally invasive surgery and other biomedical applications, means and methods for wireless manipulation of magnetic devices have been developed. As for example magnetically activated surgical catheters for endovascular procedures. Such magnetic devices may take the form of helical microrobots or helical endoscopic capsules that are inserted e.g. in a human body and which will be propelled under influence of a time-varying, rotating magnetic field. The time-varying, rotating magnetic field will induce a magnetic torque in the magnetic device which, due to e.g. its' helical shape, may be steered through an artery or vein. A time-varying, rotating magnetic field source will aim to generate low frequency waves, such 3 - 30HZ or 30 - 300 Hz, as these display low attenuation and may propagate more deeply in a harsh environment, such as dense tissue.

As source for the time-varying, rotating magnetic field, various types of electromagnetic actuators are employed. These may use permanent magnets or energized electromagnetic coil magnets or combinations thereof. They may be mounted on a robotic arm or other means for changing the position and orientation of the source magnetic field in relation to a magnetic device inside e.g. a human body. Either way, the source provides for a changing environment of the magnetic device that enables control over the magnetic device. In current systems, mostly pairs of Helmholtz-coils are used in order to create a homogenous field with which the magnetic device such as a microrobot will maintain alignment. Changing the orientation of the homogenous magnetic field will then induce a torque in the microrobot causing it to move. A drawback of such systems, esp. when mounted on a robotic arm, is that these are limited in the effective field, in terms of reach and size. Furthermore, these require an extensive mapping in order to control the microrobot. Firstly, the magnetic mapping describing the orientation of the microrobot in relation to the magnet, whether permanent or electro-actuated. And secondly, the robotic mapping expressing the orientation of the magnet in relation to the joint-space of the robotic arm.

## SUMMARY

It is an object of the invention disclosed herein to alleviate at least one of the drawbacks associated with the prior art.

In a first aspect the disclosure relates to a spherical electromagnetic actuator. The spherical electromagnetic actuator providing a magnetic field for remote control of a remote magnetic device, with the spherical electromagnetic actuator including a spherical permanent magnet having a center region, a housing comprising a chamber enclosing the spherical magnet and having an inlet for receiving a fluid. The spherical actuator further including three electromagnetic coil assemblies with each electromagnetic coil assembly having at least one coil and a virtual symmetry axis, and wherein the three electromagnetic coil assemblies are arranged such that the three respective symmetry axes intersect at at least one of a center region of the chamber and/or the center region of the spherical permanent magnet. The housing is arranged such that in operation the fluid causes the spherical magnet to levitate. And, in operation, the three electromagnetic coil assemblies provide a superpositioned magnetic field manipulating the orientation of the spherical magnet.

In another aspect the disclosure relates to a spherical electromagnetic actuator system, the system including a spherical electromagnetic actuator as disclosed and a control circuit including a driver and a current controller, for individual control of each of the electromagnetic coil assemblies of the spherical electromagnetic actuator.

In a further aspect the disclosure relates to a spherical electromagnetic actuator system, the system including at least two spherical electromagnetic actuators as disclosed. The system further including at least one control circuit including a driver and a current controller, for individual control of each of the electromagnetic coil assemblies of each of the spherical electromagnetic actuators.

In yet another aspect the disclosure relates to a method for controlling a time-varying, rotating magnetic field of at least one spherical electromagnetic actuator.

In yet another aspect the disclosure relates to a method for controlling a time-varying, rotating magnetic field of at least two spherical electromagnetic actuators.

Further objects, aspects, effects and details of particular embodiments of the invention are described in the following detailed description of a number of exemplary embodiments, with reference to the drawings.

BRIEF DESCRIPTION OF THE DRAWINGS

By way of example only, the embodiments of the present disclosure will be described with reference to the accompanying drawing, wherein:

FIG. 1 schematically illustrates a basic arrangement of a spherical electromagnetic actuator according to at least one example embodiment;

FIG. 2 schematically illustrates another basic arrangement of a spherical electromagnetic actuator according to at least one example embodiment;

FIG. 3 is an exploded view of a spherical electromagnetic actuator according to at least one example embodiment;

FIG. 4 shows a cross-sectional view of a chamber and bearing seat according to at least one example embodiment;

FIG. 5 schematically a control circuit for spherical electromagnetic actuator according to at least one example embodiment;

FIG. 6 illustrates a control model for a spherical electromagnetic actuator according to at least one example embodiment; and

FIG. 7 illustrates an example of a method for controlling a magnetic field of spherical electromagnetic actuator.

DETAILED DESCRIPTION

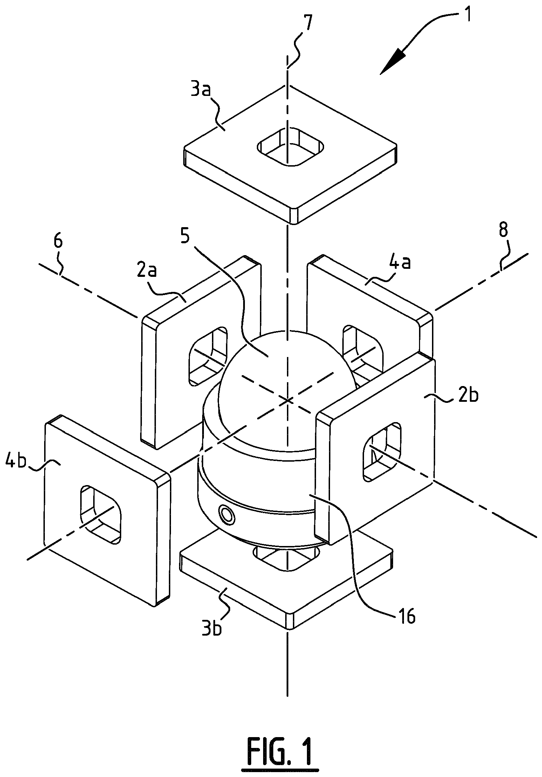

Referring to Fig. 1, an exploded view of a basic arrangement of a spherical electromagnetic actuator 1 is shown. The view displaying an arrangement of three electromagnetic coil assemblies 2a,2b; 3a,3b; 4a,4b and a spherical permanent magnet 5 having a center region. A housing 9 has a chamber 10 that may enclose the spherical permanent magnet 5. Each electromagnetic coil assembly assemblies 2a,2b; 3a,3b; 4a,4b has at least one coil and a virtual symmetry axis 6,7,8. The three electromagnetic coil assemblies 2a,2b; 3a,3b; 4a,4b are arranged such that the three respective symmetry axes 6,7,8 intersect at a center region of the spherical permanent magnet 5. A first electromagnetic coil assembly has two coils 2a, 2b which are arranged at opposite sides of the chamber 10 of the housing 9. Each coil 2a, 2b of the first coil assembly encloses a surface having a normal vector perpendicular to the enclosed surface, the normal vector co-axially aligning with the symmetry axis 6 of the first coil assembly. Hence, the symmetry axis 6 runs through the center of the coils 2a, 2b. A second electromagnetic coil assembly has two coils 3a, 3b which are arranged at opposite sides of the chamber 10 of the housing 9. Each coil 3a, 3b of the second coil assembly encloses a surface having a normal vector perpendicular to the enclosed surface, the normal vector co-axially aligning with the symmetry axis 7 of the second coil assembly. Hence, the symmetry axis 7 runs through the center of the coils 3a, 3b. A third electromagnetic coil assembly has two coils 4a, 4b which are arranged at opposite sides of the chamber 10 of the housing 9. Each coil 4a, 4b of the second coil assembly encloses a surface having a normal vector perpendicular to the enclosed surface, the normal vector co-axially aligning with the symmetry axis 8 of the third coil assembly. Hence, the symmetry axis 8 runs through the center of the coils 4a, 4b. As may be understood, the normal vectors of two coils of one coil assembly on opposite sides of the housing may also be co-axially aligned.

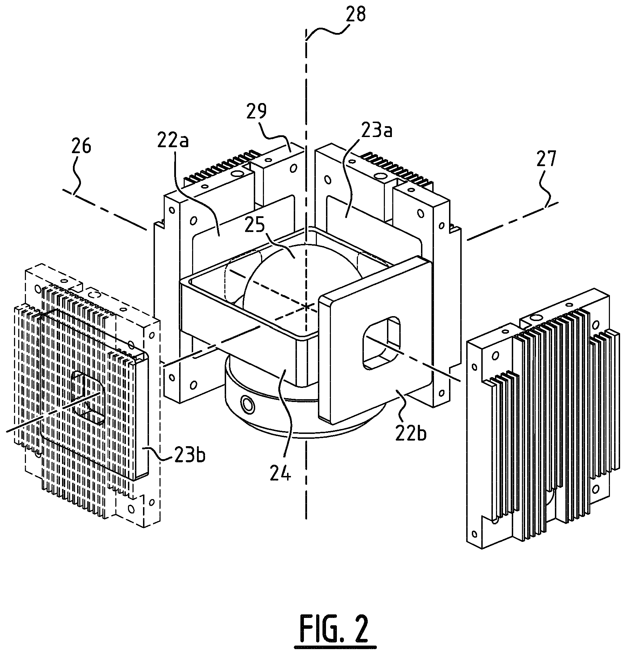

Referring to Fig.2, another basic arrangement of a spherical electromagnetic actuator 21 is shown. The view displaying another arrangement of three electromagnetic coil assemblies 22a,22b; 23a; 24 and a spherical permanent magnet 5. The housing 29 defines a chamber that may enclose the spherical permanent magnet 5. Two of the electromagnetic coil assemblies 22a,22b; 23a each have two coils arranged along respective symmetry axis 26, 28 and arranged at opposite sides of the chamber of the housing 29. Whereas, in this embodiment, one of the electromagnetic coil assemblies has a single coil 24, the single coil 24 enclosing the chamber of the housing 29. The single coil being centered about a symmetry axis 27 of the coil assembly 24.

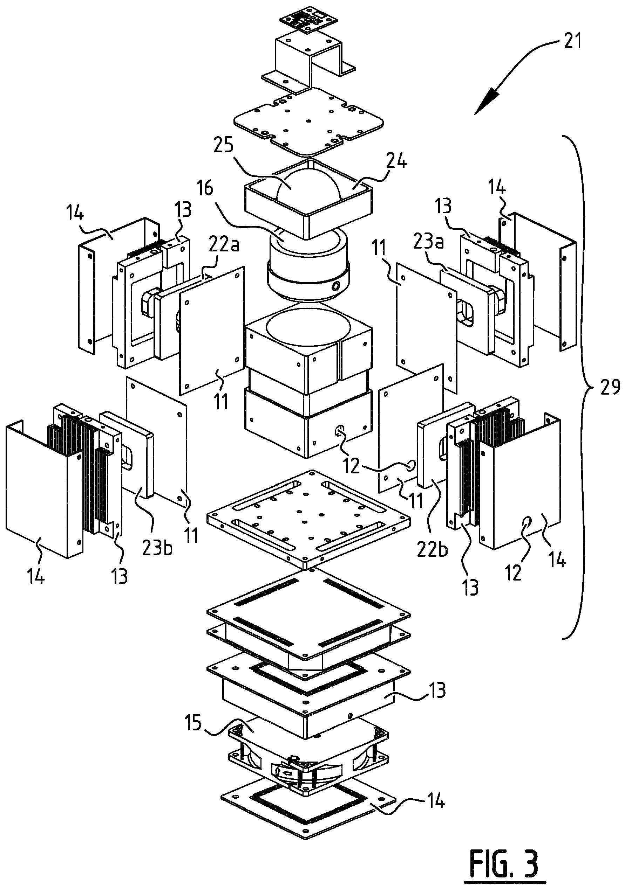

In both embodiments shown in Figs. 2 and 3, the three virtual axes 6,7,8 and 26,27,28 may intersect in the center region of the chamber 10. With the spherical permanent magnet 5 positioned within the chamber 10, the virtual axes 6, 7, 8 will substantially intersect at the center region of spherical magnet 5. The spherical magnet 5 may be made of ferromagnetic material, such as Neodymium or an alloy thereof such as Neodymium-Iron-Boron (Nd-Fe-B), also referred to as NeodymiumN. Other suitable materials may include Aluminum-Nickel, Samarium Cobalt and/or Ferrite. The size of spherical magnet may be in the order of 50 mm in diameter. The spherical magnet may have a weight of about 500 gr. The spherical magnet may have a dipole moment m between 60 - 80 A.m$^2$, and more preferably 65- 70 A.m$^2$.

Referring to Fig. 3, an exploded view of the electromagnetic actuator of Fig. 2 is shown with more detail. The housing 29 includes four walls 11 surrounding the chamber 10 and has an inlet 12 for receiving a fluid. The housing 29 is further arranged such that in operation the fluid received through the inlet 12 may act as an aerostatic bearing for the spherical magnet 5. In the example of Fig. 3, the three coil assemblies 22a,22b; 23a,23b; 24 are arranged at respective sides of the housing 29 with each coil 22a,22b; 23a,23b; 24 being in thermal connection with a respective heat sink 13. Each heat sink 13 is enclosed with a cover 14 which together with the ribs or fins of the respective heat sink 13 facilitate air flow there along. The air flow is generated by a fan 15 to cool the heat sink 13. In this manner, any heat losses of the coils may be dissipated.

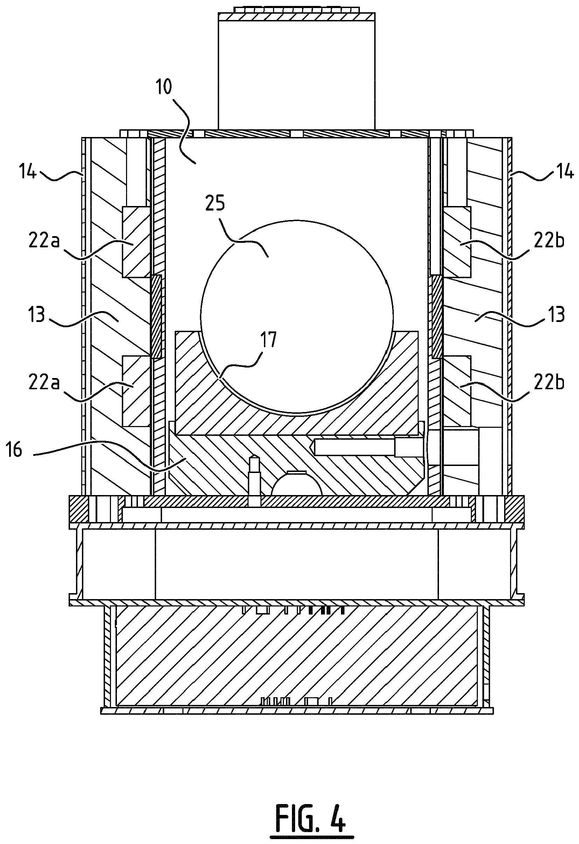

Referring to Fig. 4, the chamber 10 of the housing 9 has, in this example, a bearing seat 16. In other examples, the chamber may have an inner lining that is shaped such that it supports the sphere to stay in place. The bearing seat 16, or the inner lining, may be of porous material and therewith act as conduit for the fluid. In other embodiments, the bearing seat may be of the nozzle type, i.e. containing e.g. a plurality of nozzles and/or channels for guiding the fluid. In operation, the chamber 10 of the housing will contain the fluid 17, such as compressed air, allowing the spherical magnet 5 to rotate freely around its' center. The fluid 17 may be inserted via the fluid inlet 12. The inlet may then be sealed off, or a pressure providing device, such as a pump, may be connected for continuously keeping the fluid pressurized.

The fluid may be a gas, such as air or helium or other gas. Or the fluid may be a liquid or other suitable medium that may act as lubricant and facilitate free movement of the sphere 5 by reducing friction. The fluid preferably is compressed, alternatively it may be heated. Or it may be a combination of pressure and heat or other treatment that in operation causes the sphere to levitate within the fluid. In the case of compressed air, the fluid may be said to provide an air cushion whereon the sphere floats. More in general, a fluid may be considered as acting as aerostatic bearing when two moving parts are closely positioned and maintained contactless at a close distance from one another; such as in the example of Fig.4 the sphere and the bearing seat 16.

In operation the electromagnetic coil assemblies, as shown in Figs. 1 and 2, will provide a superimposed i.e. superpositioned magnetic field that manipulates the orientation of the spherical magnet 5, 25 and therewith also the position of its' magnetic field. Thereto, the superpositioned magnetic field of the coil pairs is to be sufficient to manipulate orientation of the spherical magnet, while the magnetic field strength of spherical magnet extends beyond that of the coil assemblies. For example, the magnetic field strength of the spherical magnet may provide a magnitude of 4mT at a distance range of 5-50 cm from the housing, and more preferably at a distance of 10 - 15 cm from the housing. As the spherical electromagnetic actuator is intended to provide a time-varying, rotating magnetic field, the actuator, with a spherical magnet having 4 mT, may be rotating at a frequency within a range of 10 - 15 Hz. thereby generating a rotating magnetic field with an average magnitude of 2 mT at a distance of 10 -15 cm.

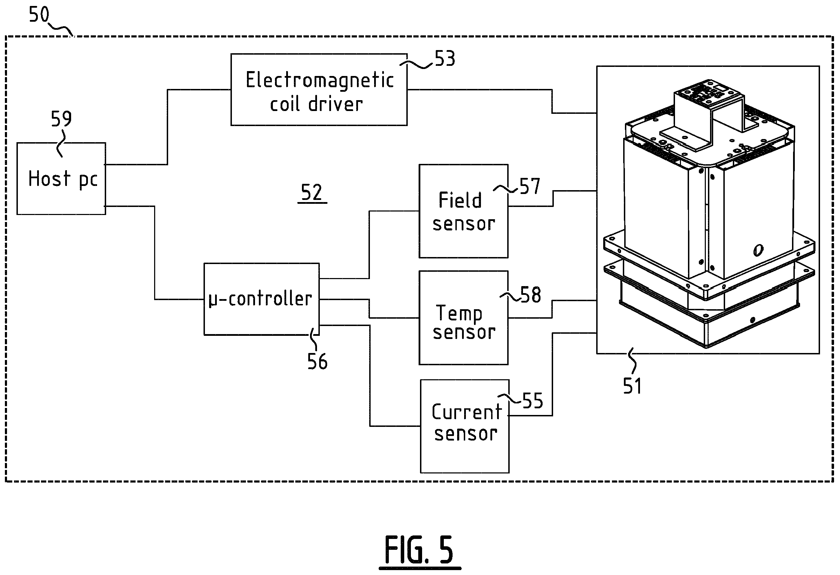

Referring to Fig. 5, an electromagnetic actuator system 50 is shown. The system includes a spherical electromagnetic actuator 51 as described above and a control circuit 52 for individual control of each of the electromagnetic coil assemblies. Thereto the control circuit 52 may include an electromagnetic coil driver 53 for delivering and controlling the currents required for the coil assemblies to operate the spherical electromagnetic actuator. The system 50 may further include a host PC 59 running a computer program enabling a user to manipulate the spherical actuator and therewith remotely control a remote magnetic device.

In order to facilitate feedback on the operation of the spherical electromagnetic actuator 51 various sensors may be included. For example, the control circuit 52 may further include at least one current sensor 55 for each coil assembly. The control circuit 52 may further include a microcontroller 56 for controlling any sensor, such as the current sensor 55, and for processing signals or values obtained by such sensor.

A spherical electromagnetic actuator system 50 including the electromagnetic actuator of the example of Fig. 1 or 2, the system may include three current sensors 55, one for each coil assembly. The current sensors 55 may be connected to the microcontroller 56 for processing the current signals. This will aid in determining the magnitude of the magnetic field that is induced by the coils and be taken as a measure for the movement of the spherical magnet.

Another type of sensor that may be included in the electromagnetic actuator system may be an array of 3D Hall sensors 57 for determining an orientation of the spherical magnet. The array of 3D Hall sensors 57 may also aid in determining a magnetization vector of the spherical magnet.

A further type of sensor that may be included in the spherical electromagnetic actuator system may be one ore more temperature sensors 58 for measuring temperature of at least one of the coils of the spherical actuator individually. This allows monitoring the operation of the actuator and ensuring that no coils overheat which may lead to improper functioning as e.g. operating outside a certain predetermined temperature range may lead to a unpredictable response of the magnetic field that is induced by the coil. For example, in one temperature range there may be a linear relation between the current in the coil and the induced magnetic field, whereas in another temperature range there may be an unpredictable non-linear response.

The spherical electromagnetic actuator system 50 is designed to remotely control at least one magnetic device, such as helical microrobot or endoscopic capsule. In order to determine where such a magnetic device is located relatively to the actuator, the system may further include a localization system for localizing the magnetic device that is to be controlled by the spherical electromagnetic actuator. Such a localization system may either be based on ultrasound, it may be based on magnetic field detection, or a combination thereof. In case of ultrasound, the localization system may include an ultrasound generator for transmitting ultrasound and an ultrasound detector for detecting deflections in the transmitted ultrasound. In use the transmitted ultrasound will be reflected by the magnetic device, which resulting deflections may be picked up by the ultrasound detector.

In order to expand the reach and maneuverability of the actuator, the spherical electromagnetic actuator system as described may further include at least one 3D motion stage, such as robotic arm or serial manipulator, to which the spherical electromagnetic actuator may be respectively mounted. When multiple 3D motion stages are used, such as e.g. with multiple robotic arms, respective spherical electromagnetic actuators may be mounted respectively upon each one of the 3D motion stages.

In order to remotely control a remote magnetic device, such as a helical microrobot, that is located at a certain distance from spherical electromagnetic actuator, it is required to provide a magnetic field displaying certain properties, viz. a time-varying, rotating magnetic field of a predetermined magnitude. Such a field will enable the helical microrobot to align and rotate with respect to its' helix axis with the magnetic field of the actuator and move forward along due to a magnetic torque. Accordingly, there is disclosed a method for controlling a time-varying, rotating magnetic field of the spherical electromagnetic actuator as described above.

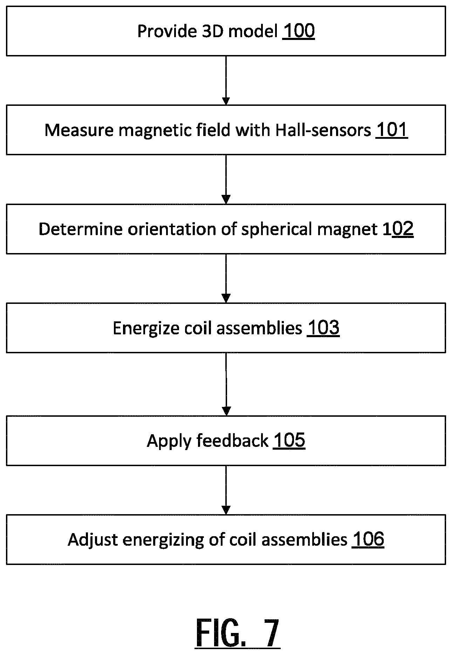

Referring to Fig. 7, a method for controlling a time-varying, rotating magnetic field of a spherical electromagnetic actuator as described is disclosed. The method includes, based on the above described models and equations, providing 100 a model of the 3-dimensional rotational motion of the spherical permanent magnet. The method further includes measuring real-time the magnetic field of the spherical magnet with an array of 3D Hall-sensors 101 and determining an orientation of the spherical magnet based on the magnetic field measurements of the Hall-sensors 102. The coil assemblies will be energized i.e. actuated by a current controller to superimpose a rotating magnetic field on the spherical magnet 103. The provided 3D model also takes in accounts an inertia induced back-emf., Based on the model feedback is provided 105 to the current controller using a exact linearization-based closed-loop current control algorithm. The actuation i.e. energizing of coil assemblies is adjusted in accordance with the provided feedback. These steps of the method may be performed repeatedly and/or continuously during the operation of the spherical electromagnetic actuator.

In the following, the development of the 3D model will be described. For a spherical magnet the magnetic field may be represented by a dipole moment m for which in relation to a 3D structure, the angular motion may be described as:

$$\mathbf{J}\dot{\omega} + d_c\omega = \tau \qquad \text{Eq. 1}$$

Herein J is an constant inertia matrix of the magnet, $d_c$ is a drag coefficient of the bearing and $\omega$ is the angular velocity of the magnet with respect to a 3D frame attached to the magnet. Furthermore, $\tau$ is the external magnetic torque generated by the coils with respect to the inertial frame of reference and is given by:

$$\tau = \mathbf{m} \times (\mathbf{q}^* \otimes \mathbf{B_c} \otimes \mathbf{q}) \qquad \text{Eq. 2}$$

Herein $B_c$ is the field generated by the coils, which is a result of the superimposed fields of the three coils, $\mathbf{q} = [q_0\ q_1\ q_2\ q_3]^T$ is the dipole orientation unit quaternion of the spherical magnet. Further $\mathbf{q}^*$ is the complex conjugate of the quaternion $\mathbf{q}$ and the $\otimes$ is the quaternion multiplication operator. The magnetic torque rotates the spherical magnet about an axis of rotation ($\hat{\omega}$) and enables the dipole moment of the spherical magnet to trail behind the continuously varying of $B_c$. Substituting Equation (2) into Equation (1) gives:

$$\mathbf{J}\dot{\omega} + d_c\omega = \mathbf{m} \times (\mathbf{q}^* \otimes \mathbf{B_c} \otimes \mathbf{q}) \qquad \text{Eq. 3}$$

The kinematic relationship between the magnet orientation quaternion and the angular velocity of the magnet can be expressed as follows

$$\dot{\mathbf{q}} = \frac{1}{2}\mathbf{q} \otimes \boldsymbol{\omega}. \qquad\qquad \text{Eq. 4}$$

Turning to the electrical model of the electromagnetic coils. An inertial frame is attached to the coils, the voltage across the $i^{th}$ coil $V_i$ is calculated using Kirchhoff's law such that:

$$L_i \dot{I}_i + R_i I_i = V_i - \varepsilon_i \qquad\qquad \text{Eq. 5}$$

Herein $L_i$ and $R_i$ are the inductance and the resistance of the $i^{th}$ coil, respectively. In Equation (5), $\varepsilon_i$ is the back-emf induced in the $i^{th}$ coil as a result of the time-varying field B generated by the rotation of the magnet. This back-emf produces an electrical current opposing the direction of the supplied current which affects the generated magnetic torque and the rotation of the magnet.

Using Faraday's law of induction, $\varepsilon_i = -N_i \left( \dfrac{\mathrm{d}\phi_i}{\mathrm{d}t} \right)$, where $\phi_i$ is the magnetic flux in relation to the inertial frame and can be written as $\phi_i = \iint_{A_i} \mathbf{B}_\mathrm{d} \cdot \mathbf{n}_i dA_i$. Herein $n_i$ is the unit vector normal to the cross-sectional area $A_i$ of the $i^{th}$ coil. In other words, the voltage induced in the square coil may be calculated by using integration of the magnetic field generated by the rotating magnet over the surface area of the coil. Therefor, $\varepsilon_i$ is determined for a square cross-sectional coil using:

$$\varepsilon_i = \frac{4\mu_0 \|\mathbf{m}\| N_i w_{0i}^2}{\pi\left(4d^2 + w_{0i}^2\right)\sqrt{4d^2 + 2w_{0i}^2}} \dot{m}_i \qquad\qquad \text{Eq. 6}$$

Herein $\omega_{0i}$ and $N_i$ are the outer-diameter and number of turns of the $i^{th}$ coil, respectively. $d$ is the center-to-center distance between the magnet and the $i^{th}$ coil and $\|\mathbf{m}\|$ describes both velocity and direction of the dipole. Equation (6) shows that the back-emf is directly proportional to the angular speed of the magnet for a given instance of geometrical parameters of the coils. The combination of both the coils and the spherical magnet generates a rotating field B(p) about an axis of rotation $\hat{\Omega}$ at point p in space. This rotating field represents the field generated by the magnetic dipole of the spherical magnet superimposed i.e. superpositioned with the field of the coil assemblies. At a relatively larger distance from the center of the coils, the field of the coils is negligible compared to that of the magnet. The field generated by the magnetic dipole is calculated by:

$$\mathbf{B(p)} = \frac{\mu_0 \|\mathbf{m}\|}{4\pi}\left( \frac{3(\hat{\mathbf{m}}\cdot\mathbf{P})\mathbf{P}}{\|\mathbf{P}\|^5} - \frac{\hat{\mathbf{m}}}{\|\mathbf{P}\|^3} \right) \qquad\qquad \text{Eq. 7}$$

Herein $\mu_0$ is the magnetic permeability of free space, P is the position vector to the point p from the center of the spherical magnet and $I \in R3\times3$ is the identity matrix.

The relation between the angular speed of the spherical magnet and the magnetic field may be expressed as follows. When the magnetic moment in the permanent magnet is displaced by an angle $\theta$ from the axis of the coils, in the direction of the field, it experiences a restoring torque that acts against the angular displacement of $\mu$, so equation 1 can be rewritten as

$$-\mathrm{m} \times \mathbf{B_c} = J\frac{\partial^2 \Theta}{\partial^2 t} + d_\mathrm{c}\frac{\partial \Theta}{\partial t} \qquad\qquad \text{Eq.8a}$$

Herein $\theta$ is the angular displacement from the direction of B. The minus sign indicates that the torque is restoring in nature. For small angle displacements $\sin\theta \approx \theta$ and in scalar form Eq.8a becomes:

$$-\mathrm{mB_c}\theta = J\frac{\partial^2 \Theta}{\partial^2 t} + d_\mathrm{c}\frac{\partial \Theta}{\partial t} \qquad\qquad \text{Eq. 8b}$$

The solution of this equation is $\Theta(t) = A_1\cos\omega t + A_2\sin\omega t$, where $\omega$ and A are constants. Plugging the value of $\Theta$ into Eq. 8b gives

$$-mB_c(A_1\cos\omega t + A_2\sin\omega t) = (J\omega^2 + d_c\,\omega)(A_1\cos\omega t + A_2\sin\omega t) \qquad Eq.\ 9$$

For this to be true for all times t, the following relation needs to be fulfilled,

$$J\omega^2 + d_c\omega + mB_c = 0 \qquad\qquad Eq.\ 10$$

The relation between the angular rotational speed of the magnet $\omega$ and the required magnetic field is given by

$$\omega = \frac{-d_c \pm \sqrt{d_c^2 - 4J\mu B_c}}{2J} \qquad\qquad Eq.\ 11$$

As an example, based on Equation (11) it may be calculated that a magnetic field of 12mT will result in a rotational speed of 750 RPM and the spherical electromagnetic actuator will provide rotating magnetic fields at a range of angular frequencies.

Based on Equation 3 the back-emf induced by inertia of the spherical actuator may be determined. Together with real-time measurements by the array of 3D Hall-sensors of the magnetic field of the spherical magnet with, the orientation of the spherical magnet may be determined.

Accordingly, the coil assemblies may be actuated by a current controller to superimpose a rotating magnetic field on the spherical magnet, while taking in account the back-emf. The provided models and obtained measurements are used to develop a linearization-based closed-loop current control algorithm. Based on the developed current control algorithm feedback may be applied to the current controller.

Continuing the development of a control law of the spherical actuator, based on the electrical model and the rotational model, conditions for linearization of the joint electrical and rotational models are developed. Which linearization will facilitate providing feedback of the orientation of the spherical magnet as reference signal for the control law.

For the case of an open-loop actuation algorithm, the orientation of the actuator magnet $\hat{m}_d$ required to generate the rotating field is given by

$$\hat{m}_d = e^{(\|\Omega\|t)\mathbb{S}(\hat{\Omega})}\hat{m}_o \qquad\qquad Eq.\ 12$$

Herein $\hat{\Omega} = \widehat{\mathbb{H}\hat{\omega}}$ is the actuator magnet rotation axis required to generate a rotating field about the axis $\hat{\omega}$ at the roller position, $\mathbb{S}(\hat{\Omega})$ is the skew-symmetric matrix of the axis $(\hat{\Omega})$. The matrix exponential of a skew-symmetric matrix creates a rotation matrix and $\hat{m}_o$ is any unit vector perpendicular to $\hat{\Omega}$ (i.e., $\hat{m}_o \perp \hat{\Omega}$). The required input currents, I, are then calculated by:

$$I = \left(\frac{2M_a R_a^2 \|\Omega\|^2}{5\mathcal{B}\|m_a\|}\right) e^{(\|\Omega\|t)\mathbb{S}(\hat{\Omega})}\hat{m}_o \qquad\qquad Eq.\ 13$$

Herein $M_a$ and $R_a$ are the mass and radius of the actuator magnet, respectively.

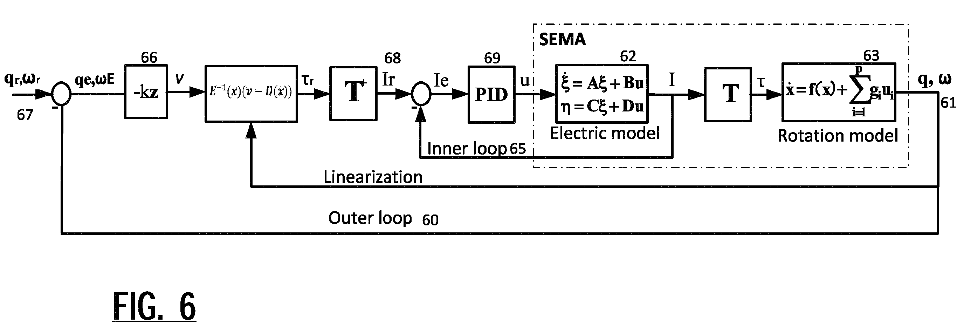

Referring to Fig.6, in order to obtain a closed-loop control model for the spherical electromagnetic actuator, in this example, a cascade control architecture is developed, which involves the use of two feedback loops. An input-output feedback linearization of the nonlinear rotation model 63 enables the use of the linear controller. The output 61 which is feedback by a first outer feedback loop 60 and compared with the reference orientation 67 enables the use of a linear "output feedback" orientation control 66. The "output feedback" orientation control 66 further provides a reference 68 for the current controller 69 using the electric model 62 of the spherical electromagnetic actuator SEMA, as shown by an inner feedback loop 65.

Starting from equations 1 and 2, the rotation dynamic model of the SEMA is given by the following set of equations:

$$\dot{q}_0 = -\frac{1}{2}\omega_x q_1 - \frac{1}{2}\omega_z q_3,$$

$$\dot{q}_1 = \frac{1}{2}\omega_x q_0 + \frac{1}{2}\omega_z q_2, \qquad J\dot{\omega}_x + d\omega_x = \frac{mb}{J}\tau_1,$$

$$\dot{q}_2 = -\frac{1}{2}\omega_z q_1 + \frac{1}{2}\omega_x q_3, \qquad J\dot{\omega}_z + d\omega_z = -\frac{mb}{J}\tau_2, \qquad \text{Eq. 14}$$

$$\dot{q}_3 = \frac{1}{2}\omega_z q_0 - \frac{1}{2}\omega_x q_2,$$

Herein,

$$\tau_1 = \left(2q_0 q_2 I_x + 2q_1 q_3 I_x - 2q_0 q_1 I_y + 2q_2 q_3 I_y + q_0^2 I_z - q_1^2 I_z - q_2^2 I_z + b I_z q_3^2\right),$$

$$\tau_2 = \left(q_0^2 I_x + I_x q_1^2 - I_x q_2^2 - I_x q_3^2 + 2q_0 q_3 I_y + 2I_y q_1 q_2 - 2q_0 q_2 I_z + 2I_z q_1 q_3\right). \qquad \text{Eq. 15}$$

Subsequently, the following state variables are defined:

$$\begin{aligned} x_1 &= q_0, & x_2 &= q_1, & x_2 &= q_2, \\ x_4 &= q_3 & x_5 &= \omega_x, & x_6 &= \omega_z. \end{aligned} \qquad \text{Eq1. 16}$$

The inputs are defined as:

$$[u_1, u_2]^T = [\tau_1, \tau_2]^T \qquad \text{Eq. 17}$$

And the outputs are defined as:

$$\begin{aligned} y_1 &= h_1(x) = m_x = -2q_3 q_0 + 2q_2 q_1 \\ y_2 &= h_2(x) = m_z = 2q_1 q_0 + 2q_2 q_3, \end{aligned} \qquad \text{Eq. 18}$$

The SEMA mechanical model is written in state-space as,

$$\begin{aligned} \dot{x} &= f(x) + \sum_{i=1}^{p} g_i(x) u_i, \\ y_i &= h_i(x), \end{aligned} \qquad \text{Eq. 19}$$

Herein, $f(x)$, $g_1(x)$ and $g_2(x)$ are defined as:

$$f(x) = \begin{bmatrix} -\frac{1}{2}x_5 x_2 - \frac{1}{2}x_6 x_4 \\ \frac{1}{2}x_5 x_1 + \frac{1}{2}x_6 x_3 \\ -\frac{1}{2}x_6 x_2 + \frac{1}{2}x_5 x_4 \\ \frac{1}{2}x_6 x_1 - \frac{1}{2}x_5 x_3 \\ \frac{-d}{J}x_5 \\ \frac{-d}{J}x_6 \end{bmatrix}, \qquad g_1(x) = \begin{bmatrix} 0 \\ 0 \\ 0 \\ 0 \\ \frac{m}{J} \\ 0 \end{bmatrix}, \qquad g_2(x) = \begin{bmatrix} 0 \\ 0 \\ 0 \\ 0 \\ 0 \\ -\frac{m}{J} \end{bmatrix} \qquad \text{Eq. 20}$$

Herein the inputs are $[u_1, u_2]^T$ and the outputs are $[y_1, y_2]^T$ as defined above. As the unit quaternion satisfies the constraint $q_0^2 + q_1^2 + q_2^2 + q_3^2 = 1$, the fourth state variable can be solved if the three other variables are known.

Still referring to Fig.6, for the input-Output Feedback linearization taking the time-derivative of the output equation yields:

$$
\begin{aligned}
y &= h(x), \\
\dot{y} &= L_f h(x), \\
\ddot{y} &= L_f^2 h(x) + L_{g_1} L_f h(x) u_1 + L_{g_2} L_f h(x) u_2
\end{aligned}
\qquad \text{Eq. 21}
$$

Therefore, the vector relative degree of the system is $[r_1 \; r_2] = [2 \; 2]$, while the dimension of the system is 6. Since $r_1 + r_2 = 4 < 6$, the nonlinear system can be input-output linearized. Thus, there following is obtained:

$$
\begin{bmatrix} y_1(r_1) \\ y_2(r_2) \end{bmatrix} = \mathbf{D}(\mathbf{x}) + \mathbf{E}(\mathbf{x})u
\qquad \text{Eq. 22}
$$

The feedback linearization is feasible if and only if the matrix $\mathbf{E}(\mathbf{x})$ is nonsingular, which means $\det(\mathbf{E}(\mathbf{x})) \neq 0$. From equations 21 and 22 it is known that:

$$
\det\big(\mathbf{E}(\mathbf{x})\big) = -\frac{m^2(x_1^4 + 2x_1^2 x_3^2 - x_2^4 - 2x_2^2 x_4^2 + x_3^4 - x_4^4)}{J^2}
\qquad \text{Eq. 23}
$$

When $\mathbf{q} \neq 0$ ($x_1$ or $x_2$ or $X_3$ or $x_4 \neq 0$), matrix $\mathbf{E}(\mathbf{x})$ is nonsingular and the input-output linearization problem is solvable. With $v = \mathbf{D}(\mathbf{x}) + \mathbf{E}(\mathbf{x})\mathbf{u}$ it can be computed that the control law takes the form of:

$$
u = \mathbf{E}^{-1}(\mathbf{x})\big(v - \mathbf{D}(\mathbf{x})\big)
\qquad \text{Eq. 24}
$$

So, using feedback linearization, the nonlinear system of equation 14 is transformed to a linear system which is within suitable coordinates controllable. Using the control law according to equation 24 yields the following linear dynamics:

$$
\begin{aligned}
\dot{z} &= \mathbf{F}z + \mathbf{H}v, \\
y &= \mathbf{S}z,
\end{aligned}
\qquad \text{Eq. 25}
$$

where,

$$
\mathbf{F} = \begin{bmatrix} \mathbf{0}_{2\times2} & \mathbf{I}_{2\times2} \\ \mathbf{0}_{2\times2} & \mathbf{0}_{2\times2} \end{bmatrix}, \qquad
\mathbf{H} = \begin{bmatrix} \mathbf{0}_{2\times2} \\ \mathbf{I}_{2\times2} \end{bmatrix}.
\qquad \text{Eq. 26}
$$

For the linearized spherical electromagnetic actuator SEMA mechanical model, a controller may be designed using a linear control law, which assigns the poles of the closed loop linear system track some desired dipole trajectory $y_d$:

$$
z_d = \begin{bmatrix} y_d & \dot{y}_d \end{bmatrix}^T
\qquad \text{Eq. 27}
$$

By letting $\tilde{z}(t) = z(t) - z_d(t)$ be the tracking error, the new input $v$ may be chosen as:

$$
\begin{aligned}
v &= \ddot{y}_d + \mathbf{k}\tilde{z}_d, \\
v &= \ddot{y}_d + k_1(\dot{y}_d - \dot{y}) + k_2(y_d - y).
\end{aligned}
\qquad \text{Eq. 28}
$$

Accordingly, based on the above developed models a control law for a control system, such as e.g. the control system of fig. 5, may be provided.

Based on Equation 6 the back-emf induced by inertia of the spherical actuator may be determined. Together with real-time measurements by the array of 3D Hall-sensors of the magnetic field of the spherical magnet with, the orientation of the spherical magnet may be determined.

Accordingly, the coil assemblies may be actuated by a current controller to superimpose a rotating magnetic field on the spherical magnet, while taking in account the back-emf. The provided models and obtained measurements are used to develop a linearization-based closed-loop current control algorithm. Based on the developed current control algorithm feedback may be applied to the current controller.

In another embodiment, a system for controlling a superpositioned time-varying, rotating magnetic field may also include two spherical electromagnetic actuators. In yet another embodiment, a system for controlling a superpositioned time-varying, rotating magnetic field may include at least two, such as three or more spherical electromagnetic actuators. The system further including at least one control circuit including a driver and a current controller, for individual control of each of the electromagnetic coil assemblies of each of the spherical electromagnetic actuators.

For a system including at least two spherical electromagnetic actuators, synchronizing the magnetic fields of the spherical electromagnetic actuators may be required. Accordingly, a method for controlling a superpositioned time-varying, rotating magnetic field of at least two spherical electromagnetic actuators is disclosed. The method includes measuring real-time the respective magnetic fields of the at least two spherical magnets with respective arrays of 3D Hall-sensors. The method further includes synchronizing the magnetization vector of the at least two spherical magnets by applying a closed-loop current control algorithm. A proportional-integralderivative PID closed -loop current control algorithm may be used therefor.

According to another embodiment, a spherical electromagnetic actuator for providing a magnetic field for remote control of a remote magnetic device is described. The spherical electromagnetic actuator including a spherical permanent magnet, a housing comprising a chamber enclosing the spherical magnet and having an inlet for receiving a fluid. The actuator further including three electromagnetic coil assemblies arranged along three respective intersecting virtual axes, wherein each coil pair is arranged such that the intersecting virtual axes intersect the center region of the spherical magnet. Herein each coil pair comprises two coils arranged on opposite sides of the housing and centered along one of the respective virtual axes. And the housing is arranged such that in operation the fluid causes the spherical magnet to levitate. During i.e. in operation the electromagnetic coil assemblies provide a superpositioned magnetic field manipulating the orientation of the spherical magnet.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

Furthermore, although exemplary embodiments have been described above in some exemplary combination of components and/or functions, it should be appreciated that, alternative embodiments may be provided by different combinations of members and/or functions without departing from the scope of the present disclosure. In addition, it is specifically contemplated that a particular feature described, either individually or as part of an embodiment, can be combined with other individually described features, or parts of other embodiments.

**Claims**

1. A spherical electromagnetic actuator for providing a magnetic field for remote control of a remote magnetic device, the actuator comprising:

   a spherical permanent magnet having a center region;
   a housing comprising a chamber (10) enclosing the spherical magnet (5), and having an inlet (12) for receiving a fluid (17);
   three electromagnetic coil assemblies (2,3,4), each electromagnetic coil assembly having at least one coil and a virtual symmetry axis (6,7,8);
   wherein the three electromagnetic coil assemblies (2,3,4) are arranged such that the three respective symmetry axes (6,7,8) intersect at at least one of a center region of the chamber (10) and/or the center region of the spherical permanent magnet (5);
   wherein the housing (9) is arranged such that in operation the fluid causes the spherical magnet to levitate;
   wherein in operation the three electromagnetic coil assemblies provide a superpositioned magnetic field manipulating the orientation of the spherical magnet.

2. The spherical electromagnetic actuator according to claim 1, wherein the coil assemblies are arranged such that the symmetry axes are orthogonal to one another

3. The spherical electromagnetic actuator of claim 1 or 2, wherein at least one coil assembly comprises at least two coils arranged on opposite sides of the housing and

wherein preferably the at least one coil of each electromagnetic coil assembly encloses a surface having a normal vector perpendicular to the enclosed surface, the normal vector co-axially aligning with the symmetry axis of the respective coil assembly; and

wherein more preferably the normal vectors of the at least two coils on opposite sides of the housing are co-axially aligned.

4. The spherical electromagnetic actuator according to any of the preceding claims, wherein the chamber of the housing comprises a bearing seat; and
wherein preferably the bearing seat comprises a porous material acting as conduit for the fluid or a plurality of nozzles; and/or .

5. The spherical electromagnetic actuator according to any of the preceding claims, further comprising a cooling arrangement, such as heat sink.

6. The spherical electromagnetic actuator according to any of the preceding claims, wherein the superpositioned magnetic field of the coil pairs is sufficient to manipulate orientation of the spherical magnet, while the magnetic field strength of spherical magnet extends beyond the magnetic field of the coil assemblies.

7. The spherical electromagnetic actuator according to any of the preceding claims, wherein the magnetic field strength of the spherical magnet provides a magnitude of 4mT at a distance range of 5-50 cm, and more preferably 10 - 15 cm from the housing.

8. The spherical electromagnetic actuator according to any of the preceding claims, wherein the spherical permanent magnet comprises Neodymium, a Neodymium-Iron-Boron alloy, Aluminum-Nickel, Samarium Cobalt and/or Ferrite.

9. An electromagnetic actuator system, comprising:

at least one spherical electromagnetic actuator according to any of the preceding claims;
and a control circuit, comprising a driver and a current controller, for individual control of each of the electromagnetic coil assemblies of the at least one spherical electromagnetic actuator.

10. The electromagnetic actuator system according to claim 9, the control circuit further comprising:

a microcontroller; and
for each coil assembly at least one current sensor for measuring current within/through the coil assembly, the current sensors being connected to the microcontroller.

11. The electromagnetic actuator system according to claim 9 or 10, further comprising an array of 3D Hall sensors for determining an orientation of the spherical magnet and/ or a magnetization vector of the spherical magnet; and/ or .
further comprising at least one temperature sensor for measuring temperature of at least one coil individually;

12. The electromagnetic actuator system according to claims 9-11, further comprising at least one magnetic device, such as helical microrobot or endoscopic capsule; and/or .

at least one 3D motion stage, such as robotic arm or serial manipulator; and
wherein preferably the at least one spherical electromagnetic actuator is mounted respectively upon one of the at least one 3D motion stage

13. The electromagnetic actuator system according to claims 9-12, further comprising a localization system for localizing a magnetic device to be controlled by the spherical electromagnetic actuator;
wherein the localization system is based on ultrasound and/or magnetic field detection.

14. Method for controlling a time-varying, rotating magnetic field of at least one spherical electromagnetic actuator according to any of claims 1-8, comprising:

providing a model of a 3-dimensional rotational motion of the spherical permanent magnet (100);

measuring real-time the magnetic field of the spherical magnet with an array of 3D Hall-sensors (101);
determining an orientation of the spherical magnet based on the magnetic field measurements of the Hall-sensors (102);
actuating by a current controller the coil assemblies to superimpose a rotating magnetic field on the spherical magnet (103);
providing feedback to the current controller using a linearization-based closed-loop current control algorithm (105);
adjusting energizing of coil assemblies in accordance with feedback (106).

15. Method for controlling a superpositioned time-varying, rotating magnetic field of at least two spherical electromagnetic actuators according to any of claims 1-8, comprising

measuring real-time the respective magnetic fields of the at least two spherical magnets with respective arrays of 3D Hall-sensors;
synchronizing the magnetization vector of the at least two spherical magnets by applying a closed-loop current control algorithm.

<u>FIG. 1</u>

FIG. 2

**FIG. 3**

## FIG. 4

FIG. 5

**FIG. 6**

Provide 3D model 100

↓

Measure magnetic field with Hall-sensors 101

↓

Determine orientation of spherical magnet 102

↓

Energize coil assemblies 103

↓

Apply feedback 105

↓

Adjust energizing of coil assemblies 106

FIG. 7

Europäisches
Patentamt

European
Patent Office

Office européen
des brevets

# EUROPEAN SEARCH REPORT

Application Number

EP 22 17 4702

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 481 158 A (ANKON MEDICAL TECH SHANGHAI CO LTD) 4 August 2020 (2020-08-04) | 1-13 | INV. A61B1/00 H02K26/00 |
| Y | * paragraphs [0002], [0037], [0040], [0041], [0072], [0076], [0096], [0100], [0101], [0103], [0111]; figures 1-4 * | 14,15 | ADD. H02K41/03 A61B34/00 H02K11/215 |
| Y | US 2016/143514 A1 (MAHONEY ARTHUR W [US] ET AL) 26 May 2016 (2016-05-26) | 14,15 | H02K11/25 H02K11/27 |
| A | * paragraphs [0004], [0005], [0028], [0038], [0041], [0047]; figures 3B, 5A * | 11 | H02K11/30 |
| A | US 2020/409475 A1 (XIA CHIYUN [US] ET AL) 31 December 2020 (2020-12-31) * paragraph [0064] * | 11 | |
| A | JP 6 760103 B2 (TOYOTA MOTOR CORP) 23 September 2020 (2020-09-23) * paragraph [0017] * | 11 | |
| A | US 2022/061642 A1 (PARK JONGOH [KR] ET AL) 3 March 2022 (2022-03-03) * paragraphs [0082] – [0089]; figure 8A * | 13 | TECHNICAL FIELDS SEARCHED (IPC) A61B H02K |
| A | EP 3 797 670 A1 (OVESCO ENDOSCOPY AG [DE]) 31 March 2021 (2021-03-31) * paragraphs [0033], [0037]; figure 1 * | 13 | |
| Y | CN 113 940 612 A (GUANGZHOU SILICON BASED INTELLIGENT CONTROL TECH CO LTD) 18 January 2022 (2022-01-18) * paragraphs [0161], [0163]; figures 1-3 * | 15 | |

The present search report has been drawn up for all claims

| Place of search | Date of completion of the search | Examiner |
|---|---|---|
| The Hague | 27 October 2022 | Georgopoulos, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 4702

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2022

| Patent document cited in search report | | Publication date | Patent family member(s) | | Publication date |
|---|---|---|---|---|---|
| CN 111481158 | A | 04-08-2020 | NONE | | |
| US 2016143514 | A1 | 26-05-2016 | US | 2016143514 A1 | 26-05-2016 |
| | | | WO | 2014201260 A1 | 18-12-2014 |
| US 2020409475 | A1 | 31-12-2020 | US | 10824245 B1 | 03-11-2020 |
| | | | US | 2020409475 A1 | 31-12-2020 |
| JP 6760103 | B2 | 23-09-2020 | JP | 6760103 B2 | 23-09-2020 |
| | | | JP | 2018121422 A | 02-08-2018 |
| US 2022061642 | A1 | 03-03-2022 | US | 2022061642 A1 | 03-03-2022 |
| | | | WO | 2020171446 A1 | 27-08-2020 |
| EP 3797670 | A1 | 31-03-2021 | CN | 114585290 A | 03-06-2022 |
| | | | EP | 3797670 A1 | 31-03-2021 |
| | | | WO | 2021058601 A1 | 01-04-2021 |
| CN 113940612 | A | 18-01-2022 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Development of Technology and Commercialization for Medical Micro-robots. Common Basis Technology Development Center for Commercialization of Medical Micro-robots. Korea Institute of Medical Microrobotics, 12 June 2019